(19) Europäisches Patentamt
European Patent Office
Office européen des brevets

(11) Veröffentlichungsnummer: **0 431 544 A2**

# (12) EUROPÄISCHE PATENTANMELDUNG

(21) Anmeldenummer: **90123155.5**

(22) Anmeldetag: **04.12.90**

(51) Int. Cl.5: **C07C 69/732**, C07C 67/343, C07C 67/03

(30) Priorität: **08.12.89 DE 3940572**

(43) Veröffentlichungstag der Anmeldung: **12.06.91 Patentblatt 91/24**

(84) Benannte Vertragsstaaten: **DE FR GB IT**

(71) Anmelder: **HOECHST AKTIENGESELLSCHAFT**
**Postfach 80 03 20**
**W-6230 Frankfurt am Main 80(DE)**

(72) Erfinder: **Schmailzl, Georg, Dr.**
**Hans-Fischer-Strasse 4**
**W-8906 Gersthofen(DE)**
Erfinder: **Wiedemann, Josef, Dr.**
**Hans-Fischer-Strasse 4**
**W-8906 Gersthofen(DE)**
Erfinder: **Pfahler, Gerhard, Dr.**
**Karlsbader Strasse 27**
**W-8900 Augsburg(DE)**
Erfinder: **Nowy, Günther, Dr.**
**Luisenruhweg 13**
**W-8901 Aystetten(DE)**

## (54) Verfahren zur Herstellung eines Bis[3,3-bis(4-hydroxy-alkylphenyl)-butansäure]diolesters.

(57) Durch die Aufteilung der bekannten Kondensation von Acetessigsäurediolestern mit Phenolen in zwei Verfahrensschritte läßt sich eine Verbilligung des Gesamtverfahrens erreichen.

Acetessigsäuremethylester wird zunächst mit einem Phenol kondensiert und der entstandene 3,3-Bis(4-hydroxy-alkylphenyl)butansäuremethylester mit dem gewünschten Diol in Gegenwart eines Umesterungskatalysators umgesetzt. Auf diese Weise ist für verschiedene Endprodukte nur ein Acetessigester erforderlich. Die Endprodukte sind wertvolle Stabilisatoren für Kunststoffe, insbesondere Polyolefine.

EP 0 431 544 A2

Xerox Copy Centre

# VERFAHREN ZUR HERSTELLUNG EINES BIS[3,3-BIS(4-HYDROXY-ALKYLPHENYL)-BUTANSÄURE]DIOLESTERS

Die vorliegende Erfindung bezieht sich auf ein Verfahren zur Herstellung eines Bis[3,3-bis(4-hydroxyalkylphenyl)-butansäure]diolesters durch Kondensation eines Acetessigesters mit einem Phenol und Umsetzung des Kondensationsproduktes mit einem Alkohol.

Bis[3,3-bis(4-hydroxy-alkylphenyl)-butansäure]diolester sind wichtige Stabilisatoren für Kunststoffe, insbesondere für Polyolefine. Sie werden hergestellt durch Kondensation von Phenolen der Formel

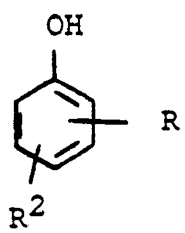

mit Acetessigestern der Formel

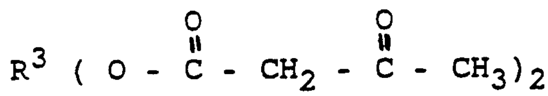

bei einer Temperatur von -10 bis +15 °C in Gegenwart von gasförmigem Chlorwasserstoff und einem $C_8$-$C_{20}$-Alkylmerkaptan (vgl. US 4 022 819). Als Ausgangsstoffe werden Acetessigsäureester von Diolen eingesetzt und die Reaktionsprodukte werden aus aromatischen Lösemitteln umkristallisiert. Für jedes Endprodukt muß ein anderer Acetessigester eingesetzt werden.

Es bestand die Aufgabe, ein Verfahren zu finden, welches einfacher durchzuführen ist und eine vergleichbare Ausbeute liefert.

Es wurde gefunden, daß die Aufgabe gelöst werden kann, wenn das Verfahren zweistufig durchgeführt wird, wobei in der ersten Stufe Acetessigsäuremethylester verwendet wird, der in der zweiten Stufe mit dem vorgesehenen Diol umgeestert wird.

Die Erfindung betrifft somit ein Verfahren zur Herstellung eines Bis[3,3-bis(4-hydroxy-alkylphenyl)-butansäure]diolesters durch Umsetzung eines Acetessigesters mit einem Phenol in Gegenwart von gasförmigem Chlorwasserstoff und einem $c_8$-$c_{20}$-n-Alkylmerkaptan, dadurch gekennzeichnet, daß zunächst Acetessigsäuremethylester mit einem Phenol der Formel I

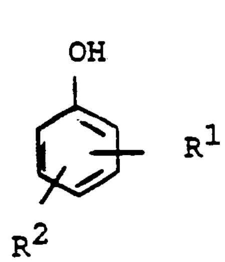

(I),

worin $R^1$ und $R^2$ gleich oder verschieden sind und ein Wasserstoffatom oder einen Alkylrest mit 1 bis 4 C-Atomen bedeuten, bei einer Temperatur von -10 bis +15 °C in Gegenwart von 5 bis 30 Gew.-%, bezogen auf den Ester, eines n-Alkylmerkaptans mit 8 bis 20 C-Atomen umgesetzt wird und der gebildete 3,3-Bis(4-hydroxy-alkylphenyl)-butansäuremethylester durch Umesterung mit einem Diol in Gegenwart von 0,05 bis 2 mol-%, bezogen auf den Ester, einer metallorganischen Verbindung der Formel II

$M(OR^3)_4$ (II),

worin $R^3$ einen Alkylrest mit 1 bis 18 C-Atomen, Phenyl oder Benzyl und M ein Metall der Gruppen IVA und IVB des Periodensystems bedeuten, oder einer Dialkylzinnverbindung der Formel III

$R^4_2$ SnO (III),

worin $R^4$ einen Alkylrest mit 4 bis 12 C-Atomen bedeutet, bei einer Temperatur von 120 bis 240 °C und einem Druck von 2 bis 300 mbar in den Bis[3,3-bis(4-hydroxy-alkylphenyl)butansäure]diolester überführt wird, den man durch Destillation bei einer Temperatur von 220 bis 280 °C und einem Druck von 0,2 bis 8 mbar von allen destillierbaren Anteilen befreit.

Als Ausgangsprodukt für das erfindungsgemäße Verfahren wird Acetessigsäuremethylester eingesetzt. Dieser Ester kann durch Anlagerung von Diketen an Methanol in Gegenwart einer basischen Verbindung, beispielsweise Triethylamin, als Katalysator hergestellt werden.

Der Ester wird in der ersten Stufe mit einem Phenol umgesetzt.

Als Phenole kommen vornehmlich solche in 4-Stellung unsubstituierte Phenole der Formel I

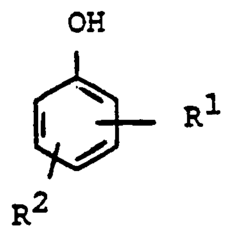

(I)

infrage, in welcher $R^1$ und $R^2$ gleich oder verschieden sind und ein Wasserstoffatom oder ein Alkylrest mit 1 bis 4 C-Atomen sein können. Genannt seien beispielsweise Phenol, o-Kresol, 2-tert.-Butylphenol, 2-Isopropylphenol, 2-Methyl-6-tert.-butylphenol und 2,6-Diisopropylphenol.

Die Reaktion wird in Gegenwart eines Merkaptans durchgeführt.

Das in einer Menge von 5 bis 30, vorzugsweise 20 bis 25 Gew.-%, bezogen auf Acetessigsäuremethylester, einzusetzende n-Alkylmerkaptan ist ein solches mit 8 bis 20, vorzugsweise 8 bis 14 C-Atomen im Molekül. Genannt seien beispielsweise n-Octylmerkaptan, n-Decylmerkaptan, n-Dodecylmerkaptan, n-Hexadecylmerkaptan und n-Octadecylmerkaptan.

Die Kondensationsreaktion wird in der Weise vorgenommen, daß man Acetessigsäuremethylester, einen Überschuß des Phenols und das Merkaptan in einem Rührbehälter vorlegt und unter Kühlung bei einer Temperatur von etwa -10 bis +15 °C trockenen Chlorwasserstoff einleitet. Nach etwa 10 bis 24, vorzugsweise 12 bis 16 Stunden ist die Kondensation beendet, was man daran sieht, daß keine exotherme Wärmetönung mehr beobachtbar ist.

Zur Erleichterung des Rührens kann der Ansatz mit einem polaren Lösemittel, wie beispielsweise Anisol oder Phenetol, verdünnt werden. Dies empfiehlt sich insbesondere gegen Ende der Umsetzung. Vorzugsweise wird in Abwesenheit eines Lösemittels gearbeitet.

Ist die Kondensationsreaktion beendet, so werden zunächst gelöster Chlorwasserstoff und gebildetes Reaktionswasser unter Erwärmen auf etwa 100 °C und Anlegen von Vakuum abgezogen. Sodann destilliert man im Vakuum das überschüssige Phenol zusammen mit dem Merkaptan und einem eventuell verwendeten Lösemittel bei einer Temperatur von 100 bis 200 °C und einem Druck von 5 bis 50 mbar ab.

Danach wird dem Ansatz ein Umesterungskatalysator zugefügt und die für das Endprodukt vorgesehene Diolkomponente eingeführt.

Als Umesterungskatalysatoren sind an sich die für diesen Zweck bekannten Verbindungen geeignet. Bevorzugt werden jedoch metallorganische Verbindungen der Metalle der Gruppen IVA und IVB des Periodensystems der Elemente der Formel II

$M(OR^3)_4$ (II),

worin M Titan, Zirkon, Hafnium, Germanium oder Zinn, vorzugsweise Titan, ist und $R^3$ einen Alkylrest mit 1 bis 18 C-Atomen, Phenyl oder Benzyl, vorzugsweise n-Butyl, bedeutet, und Dialkylzinnverbindungen der Formel III

$R^4_2$ SnO (III),

worin $R^4$ einen Alkylrest mit 4 bis 12 C-Atomen, vorzugsweise n-Butyl, bedeutet.

Der Katalysator wird in einer Menge von 0,05 bis 2 mol-%, bezogen auf das Reaktionsprodukt der ersten Stufe, eingesetzt.

Geeignete Diolkomponenten sind aliphatische, araliphatische, cycloaliphatische oder aromatische Dialkohole. Genannt seien beispielsweise Ethandiol, Propandiol-(1,2), Propandiol-(1,3), Butandiol-(1,4), Hexandiol-(1,6), Decandiol-(1,10), Dodecandiol-(1,12), 2,2-Dimethylpropandiol-(1,3), Chinit, 1,4-Dimethylolcyclohexan, Hydrochinon, Resorcin und Dioxynaphthalin. Bevorzugt sind Ethandiol und Butandiol-(1,4).

Der Alkohol wird in einer Menge von 10 bis 50 mol-%, bezogen auf das Reaktionsprodukt der ersten Stufe, eingesetzt.

Die Reaktion wird bei einer Temperatur von 120 bis 240 °C, vorzugsweise 140 bis 200 °C, bei einem Druck von 2 bis 300 mbar während 2 bis 16 Stunden durchgeführt. Zur Erleichterung des Rührens kann ein Lösemittel zugegen sein. Bevorzugt wird jedoch in Abwesenheit eines Lösemittels gearbeitet. Nach der Reaktion werden durch Vakuumdestillation alle destillierbaren Anteile entfernt. Diese Destillation wird bei einem Druck von 0,2 bis 8 mbar bei einer Temperatur von 220 bis 280 °C durchgeführt.

Zweckmäßigerweise wird in einer Kurzzeitdestillationsapparatur gearbeitet, beispielsweise in einem Dünnschichtverdampfer, Fallfilmverdampfer oder Filmtruder.

Für die meisten Anwendungszwecke besitzt der so erhaltene Bis[3,3-bis(4-hydroxy-alkylphenyl)-butansäure]diolester eine ausreichende Reinheit. Falls erforderlich, kann das Produkt jedoch zur Entfernung der Katalysatorreste aus einem Lösemittel, beispielsweise Toluol, umkristallisiert werden.

Das erfindungsgemäße Verfahren besitzt den Vorteil, daß als Ausgangsprodukt nur ein einziger Acetessigester eingesetzt wird, um verschiedene Bis[3,3-bis(4-hydroxy-alkylphenyl)butansäure]diolester herzustellen. Dadurch wird das Gesamtverfahren verbilligt. Das Endprodukt fällt in der Regel als Schmelze an, die direkt zu Pulver oder Schuppen weiterverarbeitet werden kann. Ein Lösemittel ist in der zweiten Stufe nur in Ausnahmefällen notwendig.

Das nachfolgende Beispiel soll die Erfindung erläutern.

Beispiel

In einem 1 l-Dreihalskolben mit Rührer, Gaseinleitrohr und Gasableitung wurden
348 g (2,32 mol) o-tert.-Butylphenol
46,4 g (0,40 mol) Acetessigsäuremethylester, und
16,2 g (0,08 mol) n-Dodecylmerkaptan, bezogen auf Acetessigsäuremethylester,
gegeben, worauf man in den bei einer Temperatur von 5 bis 10 °C gehaltenen Ansatz insgesamt 9,0 g trockenes HCl-Gas leitete. Nach 12 Stunden Rühren bei 5 bis 10 °C wurde im Wasserstrahlvakuum zunächst das gelöste HCl-Gas, sodann unter langsamem Steigern der Badtemperatur das gebildete Reaktionswasser und schließlich bei einer Ölbadtemperatur von 180 °C das überschüssige o-tert.-Butylphenol (Kp 9 mbar 110 °C) sowie das Dodecylmerkaptan (Kp 9 mbar 133 °C abdestilliert.

Zu dem erhaltenen 3,3-Bis(3-t-butyl-4-hydroxy-phenyl)butansäuremethylester wurden 0,5 g (0,002 mol) Dibutylzinnoxid und 11,2 g (0,18 mol) Ethandiol gegeben und der Ansatz unter Stickstoff und Rühren 2 Stunden auf 180 °C erhitzt. Dabei destillierte das entstehende Methanol ab. Anschließend wurde innerhalb 1 Stunde der Druck auf 20 mbar erniedrigt und bei diesem Druck der Ansatz weitere 4 Stunden gerührt. Danach wurde die Schmelze in einen Dünnschichtverdampfer überführt und bei einer Temperatur von 240 °C und einem Druck von 1 mbar destilliert. Der nicht umgesetzte, in der Vorlage aufgefangene 3,3-Bis-(3-t-butyl-4-hydroxyphenyl)butansäuremethylester kann wieder als Edukt verwendet werden. Die zurückbleibende Schmelze des Bis[3,3-bis(3-t-butyl-4-hydroxyphenyl)butansäure]ethandiolesters wurde unter Stickstoff abgekühlt und danach zerkleinert.

Ausbeute 135 g (0,17 mol), entspricht 94 % d.Th., bezogen auf Ethandiol, oder 85 % d.Th., bezogen auf eingesetzten Acetessigsäuremethylester.
Smp. 98 bis 110 °C.

**Ansprüche**

1. Verfahren zur Herstellung eines Bis[3,3-bis(4-hydroxy-alkylphenyl)-butansäure]diolesters durch Umsetzung eines Acetessigesters mit einem Phenol in Gegenwart von gasförmigem Chlorwasserstoff und einem $C_8$-$C_{20}$-n-Alkylmerkaptan, dadurch gekennzeichnet, daß zunächst Acetessigsäuremethylester mit einem Phenol der Formel I

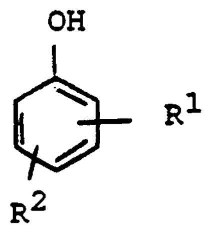

(I),

worin $R^1$ und $R^2$ gleich oder verschieden sind und ein Wasserstoffatom oder einen Alkylrest mit 1 bis 4 C-Atomen bedeuten, bei einer Temperatur von -10 bis +15 °C in Gegenwart von 5 bis 30 Gew.-%, bezogen auf den Ester, eines n-Alkylmerkaptans mit 8 bis 20 C-Atomen umgesetzt wird und der gebildete 3,3-Bis(4-hydroxy-alkylphenyl)-butansäuremethylester durch Umesterung mit einem Diol in Gegenwart von 0,05 bis 2 mol-%, bezogen auf den Ester, einer metallorganischen Verbindung der Formel II

$M(OR^3)_4$ (II),

worin $R^3$ einen Alkylrest mit 1 bis 18 C-Atomen, Phenyl oder Benzyl und M ein Metall der Gruppen IVA und IVB des Periodensystems bedeuten, oder einer Dialkylzinnverbindung der Formel III

$R^4_2 SnO$ (III),

worin $R^4$ einen Alkylrest mit 4 bis 12 C-Atomen bedeutet, bei einer Temperatur von 120 bis 240 °C und einem Druck von 2 bis 300 mbar in den Bis[3,3-bis(4 -hydroxy-alkylphenyl)butansäure]diolester überführt wird, den man durch Destillation bei einer Temperatur von 220 bis 280 °C und einem Druck von 0,2 bis 8 mbar von allen destillierbaren Anteilen befreit.

2. Verfahren nach Anspruch 1, dadurch gekennzeichnet, daß man als Phenol o-tert.-Butylphenol und als Diol Ethandiol einsetzt.

3. Bis[3,3-bis(3-t-butyl-4-hydroxyphenyl)butansäure]ethandiolester, hergestellt nach dem Verfahren gemäß Anspruch 1.

4. Verwendung des Bis[3,3-bis(3-t-butyl-4-hydroxyphenyl)butansäure]ethandiolesters nach Anspruch 3 zum Stabilisieren von Polyolefinen.